# EUROPÄISCHE PATENTANMELDUNG

(11) **EP 2 145 872 A1**
(43) Veröffentlichungstag der Anmeldung: **20.01.2010**
(21) Anmeldenummer: 08013044.6
(22) Anmeldetag: 19.07.2008
(51) Int. Cl.: C07C 45/52, C07C 47/22

(54) **Verfahren zur Dehydratisierung von Alkoholen**

(71) Anmelder: Cognis IP Management GmbH, 40589 Düsseldorf (DE)
(72) Erfinder: Schlumbohm, Carola, 40721 Hilden (DE); Wiesenhoefer, Wolfgang, 40885 Ratingen (DE); Jepkens, Daniel, 41066 Mönchengladbach (DE); Rößler, Harald, 40593 Düsseldorf (DE)

(57) **Zusammenfassung**

Die Erfindung betrifft ein Verfahren zur Dehydratisierung von Alkoholen, wobei man organische Verbindungen (E), die ein oder mehrere OH-Gruppen pro Molekül enthalten, in Gegenwart eines flüssigen Mediums und in Gegenwart eines sauren Katalysators (K) bei einer Temperatur im Bereich von 180 bis 280 °C dehydratisiert, mit der Maßgabe, dass es sich bei dem flüssigen Medium um eine ionische Flüssigkeit (IL) handelt, deren anionischer Teil durch einen Fettalkoholsulfat-, Fettalkoholethersulfat-oder Sulfonat-Baustein gekennzeichnet ist und die erhältlich ist durch Umsetzung der entsprechenden ionische Flüssigkeit (IL*), die denselben kationischen Baustein enthält, jedoch als anionischen Baustein ein Halogenid-Anion aufweist, mit einer Verbindung, die ausgewählt ist aus der Gruppe (i) der sauren Halbester eines Fettalkoholsulfats oder eines Fettalkoholethersulfats und (ii) der Sulfonsäuren, wobei man den bei der Reaktion entstehenden Halogenwasserstoff in Gasform aus dem Reaktionsgemisch entfernt.

## Beschreibung

### Gebiet der Erfindung

Die Erfindung betrifft ein Verfahren zur Dehydratisierung von Alkoholen und insbesondere zur Herstellung von Acrolein.

### Stand der Technik

Acrolein (Propenal, CH₂=CH-CHO) ist eine bekannte und weitverbreitete Industrie-Chemikalie. Es sind zahlreiche Herstellverfahren für Acrolein beschrieben worden. Ein attraktiver Weg geht dabei von Glycerin aus, das aus natürlichen Quellen in breitem Maße zur Verfügung steht. Die Bruttogleichung hierfür lautet wie folgt:

CH₂(OH)-CH(OH)-CH₂(OH) → CH₂=CH-CHO + 2 H₂O

Die Dehydratisierung von Glycerin zu Acrolein kann beispielsweise durch Reaktion in der Gasphase erfolgen. Hierfür sind Temperaturen im Bereich von 200 bis 500 °C erforderlich, vergl. z.B. US 1916743 (1933) and also US Chemicals Inc. in US 2558520 (1951), des weiteren die Gegenwart eines heterogenen Katalysators, wobei es sich in der Regel um saure Katalysatoren wie Phosphorsäure oder Aluminiumoxid handelt.

In einer jüngeren Patentanmeldung (WO 2006/092272) wird eine Kombination von Gasphasen- und Flüssigphasen-Reaktion beschrieben, wobei als Katalysator Phosphorsäure eingesetzt wird, die auf einem festen Substrat aufgebracht ist.

Aus Römpps Chemielexikon (vergl. Band 1, S. 48, 7. Auflage, Stuttgart 1972) ist bekannt, dass Acrolein u.a. beim Erhitzen von Glycerin mit wasserentziehenden Mitteln wie Kaliumhydrogensulfat entsteht. Nachteilig an dieser Verfahrensweise ist, dass die Handhabung des heterogenen Katalysators (der Katalysator ist ein Feststoff) schwierig ist. Die Salze müssen in der Regel in die Form einer Schmelze (bei Temperaturen oberhalb von 180 °C) überführt werden und tendieren im übrigen dazu, im Laufe der Reaktion miteinander wieder zu festen Aggregaten zu verkleben. Das führt zu unerwünschten Abscheidungen im Reaktor, ferner dazu, dass der Katalysator nicht recycliert oder für mehr als einen Ansatz (Batch) verwendet werden kann. Ferner treten in der Regel Probleme bei der Wärmeübetragung auf.

### Beschreibung der Erfindung

Aufgabe der vorliegenden Erfindung war es, ein Verfahren zur Dehydratisierung von Alkoholen und insbesondere ein verbessertes Verfahren zur Herstellung von Acrolein durch Dehydratisierung von Glycerin bereitzustellen, die die Nachteile des bekannten Standes der Technik vermeidet. Insbesondere sollte ein Verfahren entwickelt werden, das die Recyclierung des eingesetzten Katalysators erlaubt und das eine gute Kontrolle des Wärmeaustausches während der Prozessführung erlaubt, um auf diese Weise unerwünschte Nebenreaktionen zu minimieren.

Gegenstand der vorliegenden Erfindung ist ein Verfahren zur Dehydratisierung von Alkoholen, **dadurch gekennzeichnet, dass** man organische Verbindungen (E), die ein oder mehrere OH-Gruppen pro Molekül enthalten, in Gegenwart eines flüssigen Mediums und in Gegenwart eines sauren Katalysators (K) bei einer Temperatur im Bereich von 180 bis 280 °C dehydratisiert, mit der Maßgabe, dass es sich bei dem flüssigen Medium um eine ionische Flüssigkeit (IL) handelt. Die Natur der einzusetzenden organische Verbindungen (E), des sauren Katalysators (K) und der ionische Flüssigkeit (IL) wird in den nachfolgenden Abschnitten näher erläutert.

### Zu den organischen Verbindungen (E)

Als Edukte (E), d.h. als zu dehydratisierende Substanzen, werden im Rahmen des erfindungsgemäßen Verfahrens organische Verbindungen eingesetzt, die ein oder mehrere OH-Gruppen pro Molekül enthalten. Gewünschtenfalls können die Verbindungen (E) neben den OH-Gruppen auch ein oder mehrere weitere (d.h. von OH-Gruppen verschiedene) funktionelle Gruppen enthalten; ein Beispiel hierfür wäre Milchsäure.

In einer bevorzugten Ausführungsform enthalten die Verbindungen (E) ausschließlich OH-Gruppen als funktionelle Gruppen. Vorzugsweise werden hierbei Alkohole mit 1 bis 6 OH-Gruppen und insbesondere mit 1 bis 3 OH-Gruppen pro Molekül eingesetzt.

In einer besonders bevorzugten Ausführungsform werden die Verbindungen (E) ausgewählt aus der Gruppe Glycerin, 1,2-Propandiol, 1,3-Propandiol und der Fettalkohole mit 8 bis 18 C-Atomen. Ganz besonders bevorzugt werden die Verbindungen (E) ausgewählt aus der Gruppe Glycerin und 1,2-Propandiol.

### Zu den sauren Katalysatoren (K)

Im Rahmen des erfindungsgemäßen Verfahrens können an sich alle sauren Verbindungen als Katalysatoren eingesetzt werden. Dabei kann es sich um anorganische oder organische Protonensäuren handeln, aber auch um Lewissäuren oder Brönstedt-Säuren. Ausdrücklich sei festgestellt, dass die Verbindungen (K) von den Verbindungen (IL) verschieden sind, d.h. eine Verbindung, die der Klasse (IL) zuzurechnen ist, ist definitionsgemäß von den Verbindungen (K) ausgeschlossen.

Beispiele geeigneter saurer Katalysatoren (K) sind:
- Alle organischen und anorganischen Protonensäuren, wozu insbesondere gehören: die Mineralsäuren, Fettsäuren, Säuren der Formel R¹⁵-O-(CH₂-CH₂-O)ₓSO₃H und der Formel R¹⁶-SO₃H, wobei die Reste R¹⁵ und R¹⁶ unabhängig voneinander einen Alkylrest mit 1 bis 22 C-Atomen oder einen Arylrest mit 6 bis 18 C-Atomen, der gegebenenfalls mit ein oder mehreren Alkylresten mit jeweils 1 bis 18 C-Atomen substituiert sein kann, bedeuten und der Index x Null oder eine Zahl von 1 bis 20 bedeutet.
- Saure Salze, bei denen der saure Charakter durch mindestens ein dissoziatonsfähiges Proton vermittelt wird, wozu z.B. Hydrogen- sowie Dihydrogensalze gehören; besonders bevorzugt aus dieser Klasse sind Hydrogensulfate mit den Kationen Li, Na, K, Cs, Zn und Mg. Diese Salze können einzeln oder im Gemisch untereinander eingesetzt werden.
- Salze, die kein dissoziationsfähiges Proton aufweisen. Eine derartige Verbindung ist dann als saurer Katalysator (K) im Sinne der vorliegenden Erfindung einzustufen, wenn sie in wäßrigem Milieu einen sauren pH-Wert, d.h. einen pH-Wert von 6,9 oder weniger aufweist und zwar bei folgendem Testverfahren: Man gibt 1 g des zu prüfenden Salzes in ein Gefäß mit 100 ml destilliertem Wasser und rührt bei 20 °C bis zur vollständigen Löslichkeit bzw. - bei schwerer löslichen Substanzen - bis zur maximalen Löslichkeit. Anschließend misst man bei 20 °C den pH-Wert. Liegt dieser bei 6,9 oder weniger, so ist das entsprechend geprüfte Salz als saurer Katalysator im Sinne der vorliegenden Erfindung geeignet. Ein Beispiel für derartige Salze ist etwa Zinksulfat.

In einer Ausführungsform werden Säuren der Formel R¹⁵-O-(CH₂-CH₂-O)ₓSO₃H und der Formel R¹⁶-SO₃H, wobei die Reste R¹⁵ und R¹⁶ unabhängig voneinander einen Alkylrest mit 1 bis 22 C-Atomen oder einen Arylrest mit 6 bis 18 C-Atomen, der gegebenenfalls mit ein oder mehreren Alkylresten mit jeweils 1 bis 18 C-Atomen substituiert sein kann, bedeuten und der Index x Null oder eine Zahl von 1 bis 20 bedeutet, als sauren Katalysatoren eingesetzt.

Gewünschtenfalls können ein oder mehrere dieser Verbindungen in Abmischung mit ein oder mehreren Hydrogensulfaten mit den Kationen Li, Na, K, Cs, Zn und Mg eingesetzt werden; optional können diese Mischungen zusätzlich mit ein oder mehreren Sulfaten mit den Kationen Li, Na, K, Cs, Zn und Mg versetzt werden.

In einer Ausführungsform werden die sauren Katalysatoren in Abmischung mit neutralen Salzen eingesetzt. Unter neutralen Salzen werden solche verstanden, die kein dissoziationsfähiges Proton aufweisen und die im oben genannten Testverfahren einen pH-Wert von 7,0 oder höher aufweisen.

In einer Ausführungsform setzt man Mischungen von ein oder mehreren Hydrogensulfaten mit den Kationen Li, Na, K, Cs, Zn und Mg mit ein oder mehreren Sulfaten mit den Kationen Li, Na, K, Cs, Zn und Mg ein.

In einer bevorzugten Ausführungsform setzt man als sauren Katalysator eine Mischung von Kaliumhydrogensulfat (KHSO₄) und Kaliumsulfat (K₂SO₄) ein, wobei das Gewichtsverhältnis von (KHSO₄) zu Kaliumsulfat (K₂SO₄) im Bereich von 10 : 1 bis 1: 1 und insbesondere von 8 : 1 bis 2 : 1 liegt.

### Zu den ionischen Flüssigkeiten (IL)

Unter ionischen Flüssigkeiten versteht man im allgemeinen organische Salze oder deren Gemische, deren Schmelzpunkt unterhalb von 100 °C liegt. Die im Rahmen der vorliegenden Erfindung einzusetzenden ionischen Flüssigkeiten (IL) sind solche, deren anionischer Teil durch einen Fettalkoholsulfat-, Fettalkoholethersulfat- oder Sulfonat-Baustein gekennzeichnet ist und die erhältlich sind durch Umsetzung der entsprechende ionische Flüssigkeit (IL*), die denselben kationischen Baustein enthält, jedoch als anionischen Baustein ein Halogenid-Anion aufweist, mit einer Verbindung, die ausgewählt ist aus der Gruppe (i) der sauren Halbester eines Fettalkoholsulfats oder eines Fettalkoholethersulfats und (ii) der Sulfonsäuren, wobei man den bei der Reaktion entstehenden Halogenwasserstoff in Gasform aus dem Reaktionsgemisch entfernt.

Die Verbindungen aus der Gruppe (i) haben in einer bevorzugten Ausführungsform die allgemeine Formel R¹⁵-O-(CH₂-CH₂-O)ₓSO₃H und die Verbindungen der Gruppe (ii) vorzugsweise die allgemeine Formel R¹⁶-SO₃H, wobei die Reste R¹⁵ und R¹⁶ unabhängig voneinander einen Alkylrest mit 1 bis 22 C-Atomen oder einen Arylrest mit 6 bis 18 C-Atomen, der gegebenenfalls mit ein oder mehreren Alkylresten mit jeweils 1 bis 18 C-Atomen substituiert sein kann, bedeuten und der Index x Null oder eine Zahl von 1 bis 20 bedeutet.

Wie bereits gesagt sind die im Rahmen des erfindungsgemäßen Verfahrens einzusetzenden ionischen Flüssigkeiten (IL) erhältlich durch den Austausch des Halogenid-Anions einer ionischen Flüssigkeit (IL*) gegen ein Anion aus der oben genannten Gruppen (i) und (ii).

In einer Ausführungsform setzt man als Verbindungen (IL*) Verbindungen [A]⁺ [Hal]⁻ ein, wobei das Kation [A]⁺ ausgewählt ist aus der Gruppe der
- Quaternären Ammonium-Kationen der allgemeinen Formel [NR¹R²R³R⁴]⁺, wobei die Reste R¹ bis R⁴ unabhängig voneinander ausgewählt werden aus den Resten U1, U2, U3 und U4,
- Phosphonium-Kationen der allgemeinen Formel [PR⁵R⁶R⁷R⁸]+, wobei die Reste R⁵ bis R⁸ unabhängig voneinander ausgewählt werden aus den Resten U1, U2, U3 und U4,
- Imidazolium-Kationen der allgemeinen Formel 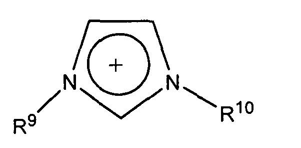
wobei die Reste R⁹ und R¹⁰ unabhängig voneinander ausgewählt werden aus den Resten Wasserstoff, U1, U2, U3, U4 und U5, mit der Maßgabe, dass nicht gleichzeitig beide Reste R⁹ und R¹⁰ Wasserstoff bedeuten, wobei gegebenenfalls der aromatische Ring des Imidazolium-Kations zusätzlich mit ein oder mehreren Resten substituiert ist, die ausgewählt sind aus den Resten U1, U2, U3, U4 und U5,
- Pyridinium-Kationen der allgemeinen Formel 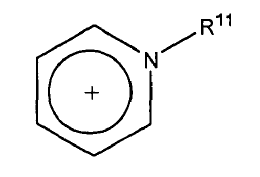
wobei der Rest R¹¹ ausgewählt wird aus den Resten Wasserstoff, U1, U2, U3, U4 und U5, wobei gegebenenfalls der aromatische Ring des Pyridinium-Kations zusätzlich mit ein oder mehreren Resten substituiert ist, die ausgewählt sind aus den Resten U1, U2, U3, U4 und U5,
- Pyrazolium-Kationen der allgemeinen Formel 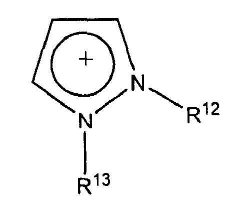
wobei die Reste R¹² und R¹³ ausgewählt wird aus den Resten Wasserstoff, U1, U2, U3, U4 und U5, mit der Maßgabe, dass nicht gleichzeitig beide Reste R⁹ und R¹⁰ Wasserstoff bedeuten, wobei gegebenenfalls der aromatische Ring des Pyrazoilum-Kations zusätzlich mit ein oder mehreren Resten substituiert ist, die ausgewählt sind aus den Resten U1, U2, U3, U4 und U5, und
- Triazolium-Kationen der allgemeinen Formel 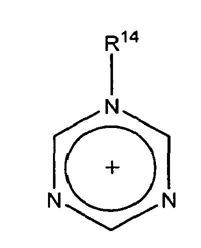
wobei der Rest R¹⁴ ausgewählt wird aus den Resten Wasserstoff, U1, U2, U3, U4 und U5, wobei gegebenenfalls der aromatische Ring des Triazolium-Kations zusätzlich mit ein oder mehreren Resten substituiert ist, die ausgewählt sind aus den Resten U1, U2, U3, U4 und U5,
wobei U1, U2, U3, U4 und U5 folgende Bedeutung haben:
- Unter **U1** ist ein aliphatischer Rest zu verstehen, der insgesamt 1-24 C-Atome aufweist. Dieser Rest kann (a) gesättigt oder ein- bzw. mehrfach olefinisch ungesättigt sein; des weiteren kann er (b) linear oder ein- bzw. mehrfach verzweigt sein und schließlich kann er (c) ein oder mehrere alicyclische Elemente aufweisen.
- Unter **U2** ist ein aromatischer Rest zu verstehen, der insgesamt 5-18 C-Atome aufweist.
- Unter **U3** ist ein ein aliphatischer Rest zu verstehen, der insgesamt 1-12 C-Atome aufweist, wobei dieser Rest gesättigt oder ein bzw. mehrfach olefinisch ungesättigt sein kann, des weiteren kann er linear oder ein- bzw. mehrfach verzweigt sein; er kann ebenfalls ein oder mehrere alicyclische Elemente aufweisen. Zwingend ist jedoch die zusätzliche Maßgabe, dass dieser aliphatische Rest durch einen aromatischen Rest mit 5 bis 18 C-Atomen substituiert ist.
- Unter **U4** ist ein aromatischer Rest zu verstehen, der insgesamt 5-18 C-Atome aufweist, wobei zwingend die Maßgabe gilt, dass dieser aromatische Rest mit ein oder mehreren aliphatischen Gruppen mit jeweils 1 bis 12 C-Atomen substituiert ist,
wobei diese aliphatischen Gruppen (a) gesättigt oder ein- bzw. mehrfach olefinisch ungesättigt sein können, (b) linear oder ein- bzw. mehrfach verzweigt sein können und (c) ein oder mehrere alicyclische Elemente aufweisen können.
- Unter **U5** ist ein Rest zu verstehen, der ausgewählt ist aus der Gruppe
   (a) -(X)ₙ-U1, wobei X Sauerstoff oder eine Gruppe NH, der Index n die Zahl 0 oder die Zahl 1 bedeutet und U1 die oben definierte Bedeutung hat,
   (b) -(X)ₙ-U2, wobei X Sauerstoff oder eine Gruppe NH, der Index n die Zahl 0 oder die Zahl 1 bedeutet und U2 die oben definierte Bedeutung hat,
   (c) -(X)ₙ-U3, wobei X Sauerstoff oder eine Gruppe NH, der Index n die Zahl 0 oder die Zahl 1 bedeutet und U3 die oben definierte Bedeutung hat,
   und der Rest [Hal]⁻ einen Halogenidrest bedeutet.

In einer besonders bevorzugten Ausführungsform setzt man als ionischen Flüssigkeiten (IL) Verbindungen der Formel (I) ein,

[NR¹⁷R¹⁸R¹⁹R²⁰]⁺ [R¹⁵-O-(CH₂-CH₂-O)ₓSO₃]⁻ (I)

worin der Rest R¹⁷ ein Benzylrest oder ein Alkylrest mit 1 bis 18 C-Atomen ist und die Reste R¹⁸ bis R²⁰ unabhängig voneinander Alkylreste mit 8 bis 18 und insbesondere 8 bis 10 C-Atomen sind, der Rest R¹⁵ einen Alkylrest mit 8 bis 22 C-Atomen und der Index x Null oder eine Zahl von 1 bis 20 bedeuten. Vorzugsweise gilt dabei, dass mindestens zwei der Alkylgruppen R¹⁷ bis R²⁰ aliphatische Reste mit Kettenlängen im Bereich von 8 bis 10 C-Atomen sind.

### Weitere Angaben zum erfindungsgemäßen Verfahren

Der Gegenstand des erfindungsgemäßen Verfahrens sowie spezielle Ausführungsformen wurden bereits oben definiert. In diesem Zusammenhang sei ausdrücklich festgestellt, dass der Begriff Dehydratisierung als Abspaltung von ein oder mehreren Molekülen Wasser pro Molekül des Eduktes (E) zu verstehen ist. Dabei ist selbstverständlich, dass beim Einsatz von Verbindungen (E), die lediglich eine OH-Gruppe pro Molekül enthalten, auch lediglich ein Molekül Wasser abgespalten werden kann, dass jedoch beim Einsatz von Verbindungen (E), die zwei oder mehrere OH-Gruppen pro Molekül enthalten, ein oder mehrere Moleküle Wasser abgespalten werden können. Wie ohne weiteres ersichtlich können bei der Dehydratisierung Aldehydgruppen, Ketogruppen, Olefingruppen und Oxirangruppen als funktionelle Gruppen entstehen, je nach der Natur des eingesetzten Eduktes (E).

In einer ganz besonders bevorzugten Ausführungsform bezieht sich das erfindungsgemäße Verfahren auf ein Verfahren zur Herstellung von Acrolein, wobei man als Verbindg (E) Glycerin einsetzt.

Das erfindungsgemäße Verfahren und insbesondere dessen Ausführungsform, das auf die Dehydratisierung von Glycerin zu Acrolein zielt, zeichnet sich insbesondere durch folgende Vorteile aus:
- Der eingesetzte saure Katalysator kann recycliert werden
- Die eingesetzte ionische Flüssigkeit kann recycliert werden
- Die Mischung aus Katalysator und ionischer Flüssigkeit bleibt beim Abkühlen flüssig; dies ist vor allem dann von Bedeutung, wenn als Katalysator ein Salz oder eine Salzmischung eingesetzt wird.
- Der Energieverbrauch ist äußerst ökonomisch
- Die Handhabung der eingesetzten Substanzen ist unkompliziert
- Es werden hohe Ausbeuten erzielt, dies gilt insbesondere für die Ausführungsform, bei der unter Einsatz von Glycerin als Verbindung (E) Acrolein hergestellt wird.
- Die Bildung von Nebenprodukten ist gering.
- Die Umsetzung erfolgt relativ schnell.
- Die Reaktion kann auch in kleinen Reaktoren durchgeführt werden, was aus Sicherheitsgründen von Vorteil ist
- Für die Ausführungsform, bei der unter Einsatz von Glycerin als Verbindung (E) Acrolein hergestellt wird, gilt: Glycerin kann in reiner oder in verdünnter Form (= in Abmischung mit Wasser) eingesetzt werden. Überraschenderweise wurden auch bei hohen Glycerin-Konzentrationen (80-100 Gew.-% Glycerin, 20-0 Gew.-% Wasser) hohe Selektivitäten für Acrolein gefunden, was vorteilhaft im Sinne der Reaktor Raum-Zeit Ausbeute ist. Die gefundenen Selektivitäten für Acrolein liegen bei bis zu 60 bis 95 % bezogen auf umgesetztes Glycerin und sind damit etwa doppelt so hoch wie Literaturangaben unter vergleichbaren Reaktionsbedingungen (Glycerinkonzentration, Temperatur, saure Dehydratisierung).

In einer bevorzugten Ausführungsform, stellt man ein Gewichtsverhältnis von ionischer Flüssigkeit zu Glycerin im Bereich von 0,015 : 1 bis 4 : 1 ein. Dabei ist ein Gewichtsverhältnis von ionischer Flüssigkeit zu Glycerin im Bereich von 0,05 : 1 bis 0,75 : 1 bevorzugt.

Das erfindungsgemäße Verfahren wird für die Ausführungsform der Dehydratisierung von Glycerin zu Acrolein, insbesondere dann, wenn man
- als ionische Flüssigkeit eine Verbindung der obigen Formel (I) einsetzt,
- die Reaktionstemperatur auf einen Wert im Bereich von 190 bis 230 °C einstellt,
- als sauren Katalysator eine Mischung von Kaliumhydrogensulfat (KHSO₄) und Kaliumsulfat (K₂SO₄) einsetzt, wobei das Gewichtsverhältnis von (KHSO₄) zu Kaliumsulfat (K₂SO₄) vorzugsweise im Bereich von 10 : 1 bis 1: 1 und insbesondere von 8 : 1 bis 2 : 1 liegt,
   vorzugsweise so durchgeführt, dass man den sauren Katalysator in die ionische Flüssigkeit einbringt, diese Mischung auf eine Temperatur im Bereich von 190 bis 230 °C bringt und dann kontinuierlich Glycerin zudosiert. Vorzugsweise wird die Reaktion dabei so durchgeführt, dass eine gleichmäßige Temperaturverteilung im Reaktor gewährleistet ist, was beispielsweise durch Einsatz eines Rührers geschehen kann.

Verfahrenstechnisch sind beim erfindungsgemäßen Verfahren Batch-, Semi-Batch- und kontinuierliche Prozessführung möglich. Hierauf wird im Folgenden eingegangen:

### Semi-Batch-Fahrweise

In einer bevorzugten Ausführungsform wird das erfindungsgemäße Verfahren, insbesondere bei der Herstellung von Acrolein aus Glycerin, als so genannte Semi-Batch-Fahrweise durchgeführt. Die Semi-Batch-Fahrweise ist in den erfindungsgemäßen Beispielen zur Herstellung von Acrolein beispielhaft dargestellt (siehe unten). Unter der Bezeichnung Semi-Batch-Fahrweise wird folgendes verstanden: Sie enthält sowohl Elemente einer reinen Batch-Fahrweise (nämlich die Vorlage von ionischer Flüssigkeit und/oder saurem Katalysator in einem Behälter) als auch solche einer kontinuierliche Fahrweise (nämlich die kontinuierliche Zufuhr der zu dehydratisierenden organischen Verbindung, z.B. Glycerin, und die kontinuierliche Entfernung des Zielproduktes, z.B. Acrolein, und von Wasser).

Vorzugsweise stellt man bei der Semi-Batch-Fahrweise zu Beginn der Reaktion, also beim Anfahren der Reaktion, ein Gewichtsverhältnis von Glycerin zu saurem Katalysator im Bereich von 0,15: 1 bis 8 : 1 und insbesondere im Bereich von 0,2 : 1 bis 3 : 1 ein; dabei ist ein Bereich von 0,2 : 1 bis 0,6 : 1 ein ganz besonders bevorzugt.

### Kontinuierliches Verfahren

In einer weiteren bevorzugten Ausführungsform wird das erfindungsgemäße Verfahren, insbesondere bei der Herstellung von Acrolein aus Glycerin, kontinuierlich durchgeführt.

Die Durchflußgeschwindigkeit kann dabei in weitem Rahmen variieren, da sie von verschiedenen Parametern wie der Reaktor-Geometrie und den gewählten Reaktionsbedingungen abhängt. Der Fachmann kann die jeweils geeignete Durchflußgeschwindigkeit im Rahmen routinemäßiger Optimierungen ermitteln.

Auch ansonsten bestehen hinsichtlich der technischen Ausgestaltung eines kontinuierlichen Prozesses keine Einschränkungen, so dass alle dem Fachmann einschlägig bekannten Konti-Technologien zum Einsatz kommen können, beispielsweise Rührkesselkaskaden.

### Weitere Gestaltungsmöglichkeiten des Verfahrens

Es ist auch möglich, das Semi-Batch Verfahren dahingehend abzuwandeln, dass man die zu dehydratisierende organischen Verbindung, z.B. Glycerin, batchweise zur ionischen Flüssigkeit und saurem Katalysator hinzugibt und lediglich das Zielprodukt, z.B. Acrolein, und Wasser kontinuierlich aus dem System entfernt.

Im übrigen kann das erfindungsgemäße Verfahren aus rein batchweise durchgeführt werden.

### Beispiele

### Eingesetzte Substanzen:

**QAV:** Quaternäre Ammoniumverbindung mit einem Wassergehalt unterhalb von 0,5 Gew.-%. Die Herstellung dieser Verbindung geschah wie folgt: 300g Aliquat 336 (Methyl-tri(octyl/decyl)-ammoniumchlorid; Handelsprodukt der Fa. Cognis) mit einem Wassergehalt von ca. 5 % Gew.-% wurden in einen Glaskolben gegeben und im Vakuum auf 50 °C erhitzt, bis der Wassergehalt unterhalb von 0,5 % Gew.-% betrug.

### Texapon N 70 - Halbester:

Schwefelsäurehalbester eines ethoxylierten C_{12/14}-Fettalkoholgemisches, erhältlich durch Sulfonierung von Dehydol LS2H (Anlagerungsprodukt von 1,85 mol Ethylenoxid an 1 mol eines Gemisches von C_{12/14}-Fettalkoholen - "Dehydol LS2H" ist ein Handelsprodukt der Firma Cognis) mit gasförmigem SO₃. Der Schwefelsäurehalbester wies eine Säurezahl von 153 auf.

### Sulfopon 12/18 - Halbester

Schwefelsäurehalbester eines C_{12/18}-Fettalkoholgemisches, erhältlich durch Sulfonierung von Lorol 12/18W (Gemisch von C_{12/18}-Fettalkoholen - "Lorol 12/18W" ist ein Handelsprodukt der Firma Cognis) mit gasförmigem SO₃. Der Schwefelsäurehalbester wies eine Säurezahl von 176 auf.

### Beispiel 1: Herstellung der ionischen Flüssigkeit IL1

140 g QAV wurden in einen Glaskolben mit Rührer gegeben. Anschließend wurden 114,46 g Texapon N 70 - Halbester zugefügt. Und die Mischung im Vakuum (Druck = 5 mbar.) bei 50 °C gerührt. Das Vakuum wurde auf 5 mbar reduziert und freigesetztes Gas (HCl) in einem Wäscher absorbiert. Diese Bedingungen wurden aufrechterhalten, bis die Gasbildung (HCl-Freisetzung) beendet war. Das Auswiegen des Rückstandes ergab 242,92 g, was auf einen vollständigen Umsatz hindeutet.

### Beispiel 2: Herstellung der ionischen Flüssigkeit IL2

110,9 g QAV wurden in einen Glaskolben mit Rührer gegeben. Anschließend wurden 82,6 g Sulfopon 12/18 - Halbester zugefügt. Und die Mischung im Vakuum (Druck = 5 mbar) bei 50 °C gerührt. Das Vakuum wurde auf 5 mbar reduziert und freigesetztes Gas (HCl) in einem Wäscher absorbiert. Diese Bedingungen wurden aufrechterhalten, bis die Gasbildung (HCl-Freisetzung) beendet war. Das Auswiegen des Rückstandes ergab 184,51 g, was auf einen vollständigen Umsatz hindeutet.

### Beispiel 3 (zum Vergleich): Herstellung von Acrolein

305 g Glycerin werden zusammen mit 1250g KHSO₄ und 250g K₂SO₄ in ein Reaktionsgefäß gefüllt und die Mischung auf 190 °C erhitzt, wobei eine exotherme Reaktion stattfand. Nach 1 Stunde klang die Exothermie ab und die Reaktion ging in eine endotherme Phase über. Nun wurden innerhalb von 270 Minuten kontinuierlich 500 g Glycerin zudosiert, wobei die Temperatur im Bereich von 200-230 °C gehalten wurde. Anschließend wurde für eine weitere Stunde in diesem Temperaturbereich von 200-230°C gehalten. Während der Reaktion wurden kontinierlich Wasser und Acrolein abdestilliert. Nach Kondensation wurde ein zweiphasiges Destillat erhalten; die als Ausbeute bilanzierte Menge an Acrolein verteilte sich dabei auf die wässrige und organische Destillatphase.

Der Umsatz von Glycerin betrug 65%, die Selektivität zu Acrolein betrug ca. 38%, so dass eine Ausbeute an Acrolein (bezogen auf die theoretische Ausbeute an Acrolein pro umgesetztem Glycerin) von 25% resultierte.

### Beispiel 4 (zum Vergleich): Herstellung von Acrolein

300 g Glycerin werden zusammen mit 1250g KHSO₄ und 250g K₂SO₄ in ein Reaktionsgefäß gefüllt und die Mischung auf 210 °C erhitzt, wobei eine exotherme Reaktion stattfand. Nach 1 Stunde klang die Exothermie ab und die Reaktion ging in eine endotherme Phase über. Nun wurden innerhalb von105 Minuten kontinuierlich 500 g Glycerin zudosiert, wobei die Temperatur bei 200-240 °C gehalten wurde. Anschließend wurde für eine weitere Stunde bei 200-220°C gehalten. Während der Reaktion wurden kontinierlich Wasser und Acrolein abdestilliert. Nach Kondensation wurde ein zweiphasiges Destillat erhalten; die als Ausbeute bilanzierte Menge an Acrolein verteilte sich dabei auf die wässrige und organische Destillatphase.

Der Umsatz von Glycerin betrug 90%, die Selektivität für Acrolein betrug 78%, womit eine Ausbeute an Acrolein von 70% (bezogen auf die theoretische Ausbeute an Acrolein pro umgesetztem Glycerin) resultierte.

### Beispiel 5 (erfindungsgemäß): Herstellung von Acrolein

750 g IL1 wurden zusammen mit 1250g KHSO₄ und 250g K₂SO₄ in ein Reaktionsgefäß gefüllt und die Mischung auf 210 °C erhitzt, wobei eine exotherme Reaktion stattfand. Nach 1 Stunde klang die Exothermie ab und die Reaktion ging in eine endotherme Phase über. Nun wurden innerhalb von 190 Minuten kontinuierlich 750 g Glycerin zudosiert, wobei die Temperatur bei 210-220 °C gehalten wurde. Anschließend wurde für eine weitere Stunde bei 215°C gehalten. Während der Reaktion wurden kontinierlich Wasser und Acrolein abdestilliert. Nach Kondensation wurde ein zweiphasiges Destillat erhalten; die als Ausbeute bilanzierte Menge an Acrolein verteilte sich dabei auf die wässrige und organische Destillatphase.

Der Umsatz von Glycerin betrug 70%, die Selektivität für Acrolein 86% womit eine Ausbeute an Acrolein (bezogen auf die theoretische Ausbeute an Acrolein pro umgesetztem Glycerin) von 60% resultierte.

### Beispiel 6 (erfindungsgemäß): Herstellung von Acrolein

750 g IL2 wurden zusammen mit 1250g KHSO₄ und 250g K₂SO₄ in ein Reaktionsgefäß gefüllt und die Mischung auf 210 °C erhitzt, wobei eine exotherme Reaktion stattfand. Nach 1 Stunde klang die Exothermie ab und die Reaktion ging in eine endotherme Phase über. Nun wurden innerhalb von 180 Minuten kontinuierlich 750 g Glycerin zudosiert, wobei die Temperatur bei 210-240 °C gehalten wurde. Anschließend wurde für eine weitere Stunde bei 210-220 °C gehalten. Während der Reaktion wurden kontinierlich Wasser und Acrolein abdestilliert. Nach Kondensation wurde ein zweiphasiges Destillat erhalten; die als Ausbeute bilanzierte Menge an Acrolein verteilte sich dabei auf die wässrige und organische Destillatphase.

Der Umsatz von Glycerin betrug 75%, die Selektivität für Acrolein 91% womit eine Ausbeute an Acrolein (bezogen auf die theoretische Ausbeute an Acrolein pro umgesetztem Glycerin) von 68% resultierte.

## Patentansprüche

1. Verfahren zur Dehydratisierung von Alkoholen, **dadurch gekennzeichnet, dass** man organische Verbindungen (E), die ein oder mehrere OH-Gruppen pro Molekül enthalten, in Gegenwart eines flüssigen Mediums und in Gegenwart eines sauren Katalysators (K) bei einer Temperatur im Bereich von 180 bis 280 °C dehydratisiert, mit der Maßgabe, dass es sich bei dem flüssigen Medium um eine ionische Flüssigkeit (IL) handelt, deren anionischer Teil durch einen Fettalkoholsulfat-, Fettalkoholethersulfat- oder Sulfonat-Baustein **gekennzeichnet** ist und die erhältlich ist durch Umsetzung der entsprechenden ionische Flüssigkeit (IL*), die denselben kationischen Baustein enthält, jedoch als anionischen Baustein ein Halogenid-Anion aufweist, mit einer Verbindung, die ausgewählt ist aus der Gruppe (i) der sauren Halbester eines Fettalkoholsulfats oder eines Fettalkoholethersulfats und (ii) der Sulfonsäuren, wobei man den bei der Reaktion entstehenden Halogenwasserstoff in Gasform aus dem Reaktionsgemisch entfernt.

2. Verfahren nach Anspruch 1, wobei die Verbindungen der Gruppe (i) die allgemeine Formel R¹⁵-O-(CH₂-CH₂-O)ₓSO₃H und die Verbindungen der Gruppe (ii) die allgemeine Formel R¹⁶-SO₃H haben, wobei die Reste R¹⁵ und R¹⁶ unabhängig voneinander einen Alkylrest mit 1 bis 22 C-Atomen oder einen Arylrest mit 6 bis 18 C-Atomen, der gegebenenfalls mit ein oder mehreren Alkylresten mit jeweils 1 bis 18 C-Atomen substituiert sein kann, bedeuten und der Index x Null oder eine Zahl von 1 bis 20 bedeutet.

3. Verfahren nach Anspruch 1 oder 2, wobei man als Verbindungen (IL*) Verbindungen [A]⁺ [Hal]⁻ einsetzt, wobei das Kation [A]⁺ ausgewählt ist aus der Gruppe der
• Quaternären Ammonium-Kationen der allgemeinen Formel [NR¹R²R³R⁴]⁺, wobei die Reste R¹ bis R⁴ unabhängig voneinander ausgewählt werden aus den Resten U1, U2, U3 und U4,
• Phosphonium-Kationen der allgemeinen Formel [PR⁵R⁶R⁷R⁸]+, wobei die Reste R⁵ bis R⁸ unabhängig voneinander ausgewählt werden aus den Resten U1, U2, U3 und U4,
• Imidazolium-Kationen der allgemeinen Formel 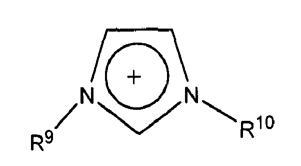
wobei die Reste R⁹ und R¹⁰ unabhängig voneinander ausgewählt werden aus den Resten Wasserstoff, U1, U2, U3, U4 und U5, mit der Maßgabe, dass nicht gleichzeitig beide Reste R⁹ und R¹⁰ Wasserstoff bedeuten, wobei gegebenenfalls der aromatische Ring des Imidazolium-Kations zusätzlich mit ein oder mehreren Resten substituiert ist, die ausgewählt sind aus den Resten U1, U2, U3, U4 und U5,
• Pyridinium-Kationen der allgemeinen Formel 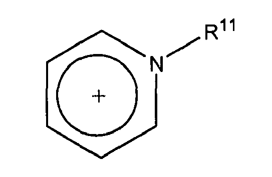
wobei der Rest R¹¹ ausgewählt wird aus den Resten Wasserstoff, U1, U2, U3, U4 und U5, wobei gegebenenfalls der aromatische Ring des Pyridinium-Kations zusätzlich mit ein oder mehreren Resten substituiert ist, die ausgewählt sind aus den Resten U1, U2, U3, U4 und U5,
• Pyrazolium-Kationen der allgemeinen Formel 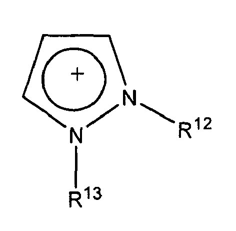
wobei die Reste R¹² und R¹³ ausgewählt wird aus den Resten Wasserstoff, U1, U2, U3, U4 und U5, mit der Maßgabe, dass nicht gleichzeitig beide Reste R⁹ und R¹⁰ Wasserstoff bedeuten, wobei gegebenenfalls der aromatische Ring des Pyrazoilum-Kations zusätzlich mit ein oder mehreren Resten substituiert ist, die ausgewählt sind aus den Resten U1, U2, U3, U4 und U5, und
• Triazolium-Kationen der allgemeinen Formel 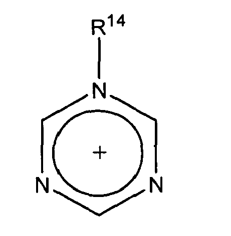
wobei der Rest R¹⁴ ausgewählt wird aus den Resten Wasserstoff, U1, U2, U3, U4 und U5, wobei gegebenenfalls der aromatische Ring des Triazolium-Kations zusätzlich mit ein oder mehreren Resten substituiert ist, die ausgewählt sind aus den Resten U1, U2, U3, U4 und U5,
wobei U1, U2, U3, U4 und U5 folgende Bedeutung haben:
• Unter **U1** ist ein aliphatischer Rest zu verstehen, der insgesamt 1-24 C-Atome aufweist. Dieser Rest kann (a) gesättigt oder ein- bzw. mehrfach olefinisch ungesättigt sein; desweiteren kann er (b) linear oder ein- bzw. mehrfach verzweigt sein und schließlich kann er (c) ein oder mehrere alicyclische Elemente aufweisen.
• Unter **U2** ist ein aromatischer Rest zu verstehen, der insgesamt 5-18 C-Atome aufweist.
• Unter **U3** ist ein aliphatischer Rest zu verstehen, der insgesamt 1-12 C-Atome aufweist, wobei dieser Rest gesättigt oder ein bzw. mehrfach olefinisch ungesättigt sein kann, des weiteren kann er linear oder ein- bzw. mehrfach verzweigt sein; er kann ebenfalls ein oder mehrere alicyclische Elemente aufweisen. Zwingend ist jedoch die zusätzliche Maßgabe, dass dieser aliphatische Rest durch einen aromatischen Rest mit 5 bis 18 C-Atomen substituiert ist.
• Unter **U4** ist ein aromatischer Rest zu verstehen, der insgesamt 5-18 C-Atome aufweist, wobei zwingend die Maßgabe gilt, dass dieser aromatische Rest mit ein oder mehreren aliphatischen Gruppen mit jeweils 1 bis 12 C-Atomen substituiert ist,
wobei diese aliphatischen Gruppen (a) gesättigt oder ein- bzw. mehrfach olefinisch ungesättigt sein können, (b) linear oder ein- bzw. mehrfach verzweigt sein können und (c) ein oder mehrere alicyclische Elemente aufweisen können.
• Unter **U5** ist ein Rest zu verstehen, der ausgewählt ist aus der Gruppe
(d) -(X)ₙ-U1, wobei X Sauerstoff oder eine Gruppe NH, der Index n die Zahl 0 oder die Zahl 1 bedeutet und U1 die oben definierte Bedeutung hat,
(e) -(X)ₙ-U2, wobei X Sauerstoff oder eine Gruppe NH, der Index n die Zahl 0 oder die Zahl 1 bedeutet und U2 die oben definierte Bedeutung hat,
(f) -(X)ₙ-U3, wobei X Sauerstoff oder eine Gruppe NH, der Index n die Zahl 0 oder die Zahl 1 bedeutet und U3 die oben definierte Bedeutung hat,
und wobei der Rest [Hal]⁻ einen Halogenidrest bedeutet.

4. Verfahren nach Anspruch 1 oder 2, wobei man als ionischen Flüssigkeiten (IL) Verbindungen der Formel (I) einsetzt
[NR¹⁷R¹⁸R¹⁹R²⁰]⁺ [R¹⁵-O-(CH₂-CH₂-O)ₓSO₃]⁻ (I)
worin der Rest R¹⁷ ein Benzylrest oder ein Alkylrest mit 1 bis 18 C-Atomen ist und die Reste R¹⁸ bis R²⁰ unabhängig voneinander Alkylreste mit 8 bis 18 sind, der Rest R¹⁵ einen Alkylrest mit 8 bis 22 C-Atomen und der Index x Null oder eine Zahl von 1 bis 20 bedeuten.

5. Verfahren nach einem der Ansprüche 1 bis 4, wobei die zu dehydratisierenden Substanzen (E) ausschließlich OH-Gruppen als funktionelle Gruppen enthalten.

6. Verfahren nach Anspruch 5, wobei man als zu dehydratisierenden Substanzen (E) Alkohole mit 1 bis 3 OH-Gruppen pro Molekül einsetzt.

7. Verfahren nach Anspruch 5, wobei man die zu dehydratisierenden Substanzen (E) auswählt aus der Gruppe Glycerin und 1,2-Propandiol.

8. Verfahren nach Anspruch 5, wobei die zu dehydratisierenden Substanzen (E) Glycerin ist und wobei das Zielprodukt Acrolein ist.

9. Verfahren nach einem der Ansprüche 1 bis 8, wobei man die Reaktionstemperatur auf einen Wert im Bereich von 190 bis 230 °C einstellt.

10. Verfahren nach einem der Ansprüche 1 bis 9, wobei man die sauren Katalysatoren (K) auswählt aus der Gruppe der organischen und anorganischen Protonensäuren, der saure Salze, bei denen der saure Charakter durch mindestens ein dissoziationsfähiges Proton vermittelt wird

11. Verfahren nach einem der Ansprüche 1 bis 9, wobei man als saure Katalysatoren (K) Säuren der Formel R¹⁵-O-(CH₂-CH₂-O)ₓSO₃H und der Formel R¹⁶-SO₃H einsetzt, wobei die Reste R¹⁵ und R¹⁶ unabhängig voneinander einen Alkylrest mit 1 bis 22 C-Atomen oder einen Arylrest mit 6 bis 18 C-Atomen, der gegebenenfalls mit ein oder mehreren Alkylresten mit jeweils 1 bis 18 C-Atomen substituiert sein kann, bedeuten und der Index x Null oder eine Zahl von 1 bis 20 bedeutet.

12. Verfahren nach Anspruch 11, wobei man als zusätzliche Katalysatoren (K) ein oder mehrere Hydrogensulfate mit den Kationen Li, Na, K, Cs, Zn und Mg einsetzt.

13. Verfahren nach einem der Ansprüche 1 bis 9, wobei man als saure Katalysatoren (K) Mischungen von ein oder mehreren Hydrogensulfaten mit den Kationen Li, Na, K, Cs, Zn und Mg mit ein oder mehreren Sulfaten mit den Kationen Li, Na, K, Cs, Zn und Mg einsetzt.

14. Verfahren nach einem der Ansprüche 1 bis 9, wobei man als saure Katalysatoren (K) eine Mischung von Kaliumhydrogensulfat (KHSO₄) und Kaliumsulfat (K₂SO₄) einsetzt, wobei das Gewichtsverhältnis von (KHSO₄) zu Kaliumsulfat (K₂SO₄) im Bereich von 10 : 1 bis 1: 1 liegt.
